# EUROPEAN PATENT APPLICATION

(11) **EP 1 136 072 A1**
(43) Date of publication of application: **26.09.2001**
(21) Application number: 99972921.3
(22) Date of filing: 02.12.1999
(51) Int. Cl.: A61K 31/475, A61P 15/00, A61P 15/10, A61P 43/00, C07D 471/06

(54) **PREPARATIONS FOR INTRAURETHRAL ADMINISTRATION**

(30) Priority: 02.12.1998 JP 34318598
(71) Applicant: MOCHIDA PHARMACEUTICAL CO., LTD., Shinjuku-ku Tokyo 160-8515 (JP)
(72) Inventor: SUZUKI, Shigeharu, Shinjuku-ku, Tokyo 160-8515 (JP); ASO, Yoshio, Fujieda-shi, Shizuoka 426-0078 (JP)
(74) Representative: Grünecker, Kinkeldey, Stockmair & Schwanhäusser Anwaltssozietät
(86) International application number: JP9906752
(87) International publication number: WO0032195

(57) **Abstract**

Pharmaceutical compositions for intraurethral administration which contain, as the active ingredient, at least one member selected from among compounds having a cyclic GMP phosphodiesterase (hereinafter referred to simply as cGMP-PDB) inhibitory effect, in particular, pyridocarbazole derivatives represented by general formula (I), salts thereof and solvates thereof. These compositions potently and selectively inhibit cGMP-PDE without being affected by drug absorption caused by foods, exert little side effects, have a high safety, and exhibit excellent therapeutic effects on erectile dysfunction and female sexual dysfunction.

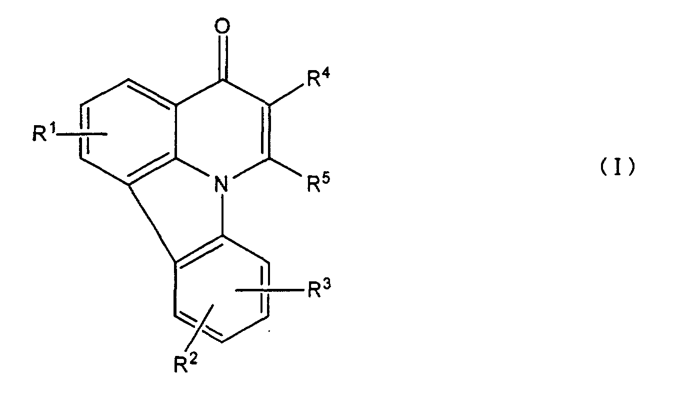

## Description

### [Technical Field]

The present invention relates to pharmaceutical compositions for intraurethral administration which potently and selectively inhibit cyclic GMP phosphodiesterase (hereinafter, abbreviated as cGMP-PDE), have a high safety, and exhibit excellent therapeutic effects on erectile dysfunction and female sexual dysfunction.

### [Background Art]

Impotence can be defined as a deficiency of ability of sexual intercourse in male and, to be more specific, impotence or erectile dysfunction can be defined as a state where hardness of penis or lasting time of erection which is sufficient for sexual intercourse cannot be achieved. Its occurring rate is said to be 2-7% of male population under 50 years old and it has been reported that the rate further increases with an increase in age. It is presumed that, in Japan, there are about three million patients of erectile dysfunction. Most of them are not psychogenic but rather organic.

In today's medical therapy, it has been reported that injection of a vasoactive substance selected from phenoxybenzamine, phentolamine, papaverine and prostaglandin E1 derivative solely or in combination into cavernous body induces good erection. However, when some of those drugs are administered into cavernous body, this is results in pain of penis, prolonged erection and corporal fibrosis. Moreover, injection into cavernous body by a patient himself/herself is not allowed in Japan whereby such a means is not practical. Potassium channel openers and vasoactive intestinal peptide are known to be active by injection into cavernous body but they have not been actually used due to a problem of cost and safety. One substitute for the administration into cavernous body is the use of nitroglycerin patch applied to penis. Although this was confirmed to be effective, it results in a side effect to both patient and partner.

On the other hand, effectiveness of pharmaceutical compositions for oral administration is low and there has been almost no report for well-controlled clinical test for human being. Recently, there is a report on the clinical test result by oral administration of 5-[2-ethoxy-5-(4-methylpiperazin-1-ylsulfonyl)phenyl]-1-methyl-3-propyl-6,7-dihydro-1H-pyrazolo[4,3-d]pyrimidin-7-one-citrate (hereinafter, abbreviated as sildenafil) (cf. Drugs of the Future, volume 22, pages 138-143, 1997). This compound has been put on the market. However, side effects such as headache, flushing, dyspepsia, muscular pain and visual disability were reported and, in addition, it has been known that absorption of the drug into body is apt to be affected by food.

Moreover, with regard to intraurethral administration, Japanese Patent Translation Publication No. Hei 4-501707 and Japanese Patent Translation Publication No. Hei 5-506800 disclose methods for the therapy of impotence by intraurethral administration of vasoactive α-blockers, prostaglandins, vasoactive intestinal polypeptide (VIP) or the like. Recently, a pharmaceutical composition, in the form of pellets containing alprostadil, a prostaglandin pharmaceutical composition, for intraurethral admistration has been developed and its effectiveness and safety have been reported (The New England Journal of Medicine, volume 336, p. 1-7 (1997)). Although its effectiveness has been confirmed, it still causes pain of penis and low blood pressure so that it cannot be said to be sufficient as a therapeutic agent for erectile dysfunction that is safe and has no side effect.

In the mechanism of erection, it is believed that a selective suppression of cGMP decomposing system also maintains the cGMP concentration to erect the penis. Thus, an inhibitor of phosphodiesterase (hereinafter, abbreviated as PDE) which is an enzyme specifically catalyzing the decomposition of cGMP is expected as a new therapeutic agent without the above-mentioned side effect. As such, it is believed that, when PDE is inhibited, cGMP increases and that might be related to the therapy. At present, existence of at least seven isozymes was ascertained for PDE (cf. Physiological Reviews, volume 75, pages 725-748, 1995). Among them, five isozymes were widely distributed in many tissues. There are two isozymes which selectively decompose cyclic GMP (hereinafter, abbreviated as cGMP) and they are PDE type I (calmodulin-dependent PDE) and PDE type V (cGMP-PDE). On the other hand, PDE type III and PDE type IV selectively decompose cyclic AMP (hereinafter, abbreviated as cAMP) and PDE type II has no substrate selectivity. It is easily predicted that, when the last three isozymes are inhibited, cAMP increases whereby various side effects such as increase in contraction of myocardium, increase in heart rate and decrease of systemic blood pressure take place. Although there is a report that, when cGMP is increased in myocardium, contraction decreases, PDE type V is not distributed in myocardium. PDE type VI exists in retina and it is expected that when this PDE is inhibited, visual disability such as bluish eyesight or hypersensitivity to light occurs. Accordingly, when PDE type V is selectively inhibited, a selective action with little lowering effect on systemic blood pressure and little side effect on heart or retina is expected.

Examples of the cGMP-PDE inhibitors disclosed up to now are pyrazolopyrimidinone derivatives in the European Patent Publication No. 463,756 or No. 526,004; purinone derivatives in the JP-A-63-196585 and 2-88577, International Publication WO 94/00,453 or the JP-A-8-231545, 8-231546 or 9-124648; quinazoline derivatives in the JP-A-52-100479, International Publication WO95/06,648 or WO 96/26,940, or JP-A-8-104679, 7-126255, 6-192235 or 7-10843; quinazolinone derivatives in JP-A-2-193983, International Publication WO 93/12,095 or JP-A-8-253457; pyrimidine derivatives in JP-A-53-103497 or 61-236778, US Patent No. 5,294,612, JP-A-2-42079, 2-56484, 2-40388, 3-261785, 7-89958, 7-267961, 7-330777 or 8-143571, US Patent No. 5,525,604 or No. 5,541,187, International Publication WO 96/28,429 or WO 96/28,448 or JP-A-8-253484; pyrimidinone derivatives in JP-A-2-295978 or International Publication WO 93/06,104, WO 93/07,149 or WO 94/05,561; grizeol derivatives in JP-A-62-30796; phenylpyridone derivatives in JP-A-1-311067 or 3-145466; dihydropyridine derivatives in JP-A-2-223580; polycyclic guanine derivatives in International Publication WO 91/19,717 or WO 94/19,351; benzimidazole derivatives in JP-A-5-222000 or International Publication WO 97/24,334; fluorenone derivatives in JP-A-7-61949; anthranilic acid derivatives in JP-A-8-188563; pyridazine derivatives in JP-A-8-225541; pyrazoloquinoline derivatives in International Publication WO 96/28,446; pyridopyrazinone derivatives in JP-A-8-269059; indole derivatives in International Publication WO 96/32,379; dihydropyrazolone derivatives in JP-A-8-311035; phthalazine derivatives in International Publication WO 96/05,176; imidazoquinazoline derivatives in the International Patent Publication WO 98/08848; or thienopyrimidine derivatives in International Publication WO 98/06722. However, there is no disclosure at all for a composition containing those compounds for the therapy of erectile dysfunction by intraurethral administration.

On the other hand, with regard to pyridocarbazole derivatives, although WO 97/45427 discloses that they have an PDE-V inhibitory action and that they are applicable to erectile dysfunction, there is no report on pharmaceutical compositions for intraurethral administration.

### [Disclosure of the Invention]

An object of the present invention is to provide a novel pharmaceutical composition for the treatment of erectile dysfunction or female sexual dysfunction that contains at least one compound having a high enzyme selectivity and potent cGMP-PDE inhibitory action as an effective component and which presents high safety with reduced side effects, particularly one that is intended for intraurethral administration.

Another object of the invention is to provide a novel pharmaceutical composition for intraurethral administration that contains at least one compound having a highly enzyme selectivity and potent cGMP-PDE inhibitory action as an effective component and which presents high safety with reduced side effects.

The present invention is particularly intended to provide pharmaceutical compositions for intraurethral administration for the treatment of erectile dysfunction and female sexual dysfunction that have solved at least one or more of the aforementioned problems in the prior art and which contain at least one compound having a high enzyme selectivity and potent cGMP-PDE inhibitory action as an effective component.

Further, the present invention is intended to provide a method for preventing or treating erectile dysfunction or female sexual dysfunction, comprising intraurethrally administering at least one of the above-mentioned pharmaceutical compositions.

Also, the present invention is intended to provide use of at least one of the above-mentioned pharmaceutical compositions in the production of a preventive or therapeutic agent for erectile dysfunction or female sexual dysfunction.

The present inventors found that when the compounds having a cGMP-PDE inhibitory action were administered intraurethrally, there were obtained more preferred absorption dynamics than when they were administered perorally, as exemplified by the avoidance of the first pass effect, reduced side effects in the digestive tract, absorption free from the effects of food, potential rapidity in action and appropriate lasting time of medicinal effect, as well as higher clinical utility than when the compounds were injected into the cavernous tissue, as exemplified by the absence of side effects such as penile pain, prolonged erection and fibrosis or systemic side effects such as low blood pressure, administrability by the patient himself with reduced burden on both the patient and the doctor. The present invention has been accomplished on the basis of this finding.

The present inventors found that more preferably, the pyridocarbazole derivatives and salts thereof represented by the formula (I) hereinbelow as compounds have potent and selective inhibitory action to PDE type V and were useful as a pharmaceutical composition for treating erectile dysfunction and a pharmaceutical composition for treating female sexual dysfunction by intraurethral administration.

The first aspect of the present invention is a pharmaceutical composition for intraurethral administration, wherein the composition contains at least one of compounds having a cyclic GMP phosphodiesterase inhibiting action, salts or solvates thereof as an effective component, in particular a pharmaceutical composition for preventing or treating erectile dysfunction or a pharmaceutical composition for preventing or treating female sexual dysfunction.

The second aspect of the present invention is a pharmaceutical composition for intraurethral administration wherein the composition contains at least one of the compounds represented by the following formula (I) and salts or solvates thereof as an effective component: 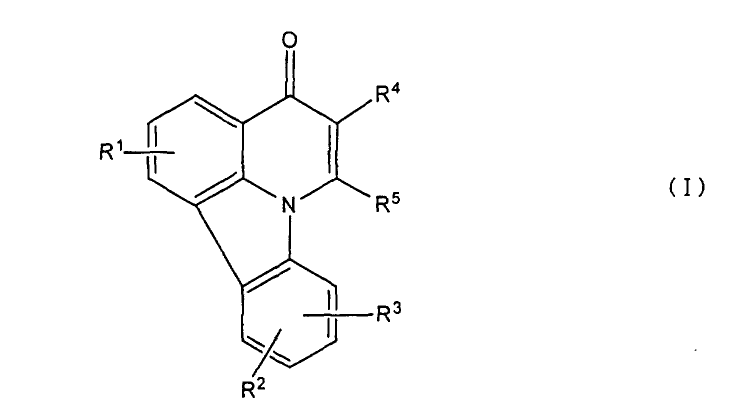 (where R¹ represents a hydrogen atom, a halogen atom, a cyano group, an optionally protected carboxyl group, an optionally protected carboxymethyl group, an alkoxycarbonyl group having 1 to 4 carbon atoms, a carbamoyl group, an acetylamino group, a 3-carboxy-1-propenyl group, a 2-hydroxypentyloxy group, a 2,2-diethoxyethoxy group, an optionally protected hydroxyl group, an optionally protected mercapto group, a straight- or branched-chain alkanoyloxy group having 1 to 4 carbon atoms, a carbonyloxy group substituted with a phenyl group or a pyridyl group, a straight- or branched-chain alkyl group having 1 to 4 carbon atoms which may be substituted with one hydroxyl group, an amino group which may be mono- or disubstituted with an alkyl group having 1 to 4 carbon atoms, an alkylthio group having 1 to 3 carbon atoms which may be monosubstituted with any group selected from the group consisting of a hydroxyl group, a carboxyl group, a phenyl group and a pyridyl group, or represented by the following formula (II):

- O - (CH₂)ₙ - Z (II)

(where Z is selected from the group consisting of a hydrogen atom, a carboxyl group, an alkoxy group having 1 or 2 carbon atoms which may be substituted with one hydroxyl group, an alkoxycarbonyl group having 1 to 6 carbon atoms, a carbamoyl group which may be mono- or disubstituted with a hydroxymethyl group or an alkyl group having 1 or 2 carbon atoms, an alkanoyl group having 1 to 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 to 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 to 6); R² is selected from the group consisting of a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a 4-morpholylacetyl group, a straight- or branched-chain alkanoyloxy group having 1 to 4 carbon atoms, a straight- or branched-chain alkanoyl group having 1 to 4 carbon atoms, a straight- or branched-chain alkyl group having 1 to 4 carbon atoms, an alkylthio group having 1 to 3 carbon atoms which may be monosubstituted with any group selected from the group consisting of a hydroxyl group, a carboxyl group, a phenyl group and a pyridyl group and a straight- or branched-chain alkoxy group having 1 to 4 carbon atoms which may be substituted with one alkoxycarbonyl group having 1 to 4 carbon atoms; R³ is selected from the group consisting of a hydrogen atom, a halogen atom, an optionally protected hydroxyl group and a straight- or branched-chain alkoxy group having 1 to 4 carbon atoms; R⁴ is selected from the group consisting of a hydrogen atom, a halogen atom, an optionally protected carboxyl group, a phenoxy group, an anilino group, a N-methylanilino group, a 4-morpholylcarbonyl group, an alkyl group having 1 or 2 carbon atoms which may be substituted with a cyclic alkyl group having 3 to 6 carbon atoms, a benzyl group which may be mono- or disubstituted in the phenyl portion with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 to 4 carbon atoms, an acetylamino group, a carboxyl group and an amino group, a pyridylmethyl group which may be substituted with an alkyl group having 1 to 4 carbon atoms, a morpholylmethyl group, a triazolylmethyl group, a furylmethyl group, a thienylmethyl group, a pyrimidinylmethyl group, a pyrazinylmethyl group, a pyrrolylmethyl group, an imidazolylmethyl group, a quinolylmethyl group, an indolylmethyl group, a naphthylmethyl group, a benzoyl group, an α-hydroxybenzyl group and an alkoxycarbonyl group having 1 or 2 carbon atoms; R⁵ represents a hydrogen atom or a methyl group; when R¹, R², R³ and R⁵ are a hydrogen atom at the same time, R⁴ is not a hydrogen atom, a benzyl group, a 4-diethylaminobenzyl group or a furylmethyl group.)

The preferred substituents in the compounds represented by the general formula (I) or the preferred combinations thereof are shown below but the invention is by no means limited thereto.

Speaking of R¹, it is preferably substituted in 2-position and is preferably a hydroxyl group or represented by the following general formula (II):

- O - (CH₂)ₙ - Z (II)

where Z represents a hydrogen atom, a carboxyl group, an alkoxycarbonyl group having 1 to 6 carbon atoms, a carbamoyl group which may be mono- or disubstituted by a hydroxymethyl group or an alkyl group having 1 or 2 carbon atoms, an alkanoyl group having 1 to 4 carbon atoms which may be substituted by one hydroxyl group or mercapto group, a hydroxyl group, an amino group, a phenyl group, a pyridyl group which may be monosubstituted by a hydroxymethyl group or an acetoxymethyl group or an alkyl group having 1 to 4 carbon atoms or an alkoxycarbonyl group having 1 or 2 carbon atoms; n is an integer of 1 to 4.

More preferably, R¹ is substituted in 2-position and is either a hydroxyl group or represented by the following general formula (II):

- O - (CH₂)ₙ - Z (II)

where Z represents a hydrogen atom, a carboxyl group, a carbamoyl group which may be mono- or disubstituted by a hydroxymethyl group or an alkyl group having 1 or 2 carbon atoms, an alkanoyl group having 1 to 4 carbon atoms which may be substituted by one hydroxyl group or mercapto group, a hydroxyl group, a phenyl group, a pyridyl group, a pyrazinyl group or a pyrimidinyl group; n is an integer of 1 to 4.

Preferably, R² and R³ are not a hydrogen atom at the same time; it is preferred that R² is substituted in 9- or 10-position and is a hydrogen atom, a halogen atom, a hydroxyl group, a trifluoromethyl group or a straight- or branched-chain alkoxy group having 1 to 4 carbon atoms and that R³ is a hydrogen atom. Further, it is more preferred that R² is substituted in 9-position and is a halogen atom or a trifluoromethyl group and that R³ is a hydrogen atom.

Preferably, R⁴ is a hydrogen atom, an alkyl group having 1 or 2 carbon atoms, a pyrimidinylmethyl group or a pyridylmethyl group which may be substituted by a methyl group. Further, it is more preferred that R⁴ is a methyl group, a pyrimidinylmethyl group or a pyridylmethyl group. Preferably, R⁵ is a hydrogen atom.

The preferred combinations of the substituents are as follows: R¹ is substituted in 2-position and is either a hydroxyl group or represented by the following general formula (II):

- O - (CH₂)ₙ - Z (II)

where Z represents a hydrogen atom, a carboxyl group, a carbamoyl group which may be mono- or disubstituted by a hydroxylmethyl group or an alkyl group having 1 or 2 alkyl groups, an alkanoyl group having 1 to 4 carbon atoms which may be substituted by one hydroxyl group or mercapto group, a hydroxyl group, a phenyl group, a pyridyl group, a pyrazinyl group or a pyrimidinyl group; n is an integer of 1 to 4; R² is a halogen atom or a trifluoromethyl group which are substituted in 9-position; R³ is a hydrogen atom; R⁴ is a methyl group, a pyrimidinylmethyl group or a pyridylmethyl group; and R⁵ is a hydrogen atom.

The particularly preferred compounds are 9-bromo-2-(3-hydroxypropyloxy)-5-(3-pyridylmethyl)-4H-pyrido[3,2,1-jk]carbazol-4-one, and salts or solvates thereof.

The third aspect of the present invention is a pharmaceutical composition for intraurethral administration for preventing or treating erectile dysfunction wherein the composition contains at least one of the compounds represented by the above formula (I) and salts or solvates thereof as an effective component.

The fourth aspect of the present invention is a pharmaceutical composition for intraurethral administration for preventing or treating female sexual dysfunction wherein the composition contains at least one of the compounds represented by the above formula (I) and salts or solvates thereof as an effective component.

In the third and fourth aspects of the present invention, the preferred substituents in the compounds represented by the general formula (I) or the preferred combinations thereof are the same as those in the second aspect.

The fifth aspect of the present invention is a pharmaceutical composition for intraurethral administration, especially a pharmaceutical composition for intraurethral administration for preventing or treating erectile dysfunction or for preventing or treating female sexual dysfunction wherein the composition contains 9-bromo-2-(3-hydroxypropyloxy)-5-(3-pyridylmethyl)-4H-pyrido[3,2,1-jk]carbazol-4-one or a salt or solvate thereof as an effective component.

The sixth aspect of the present invention is a pharmaceutical composition for intraurethral administration wherein the composition contains at least one of the compounds represented by the following formula (III) and salts or solvates thereof as an effective component: 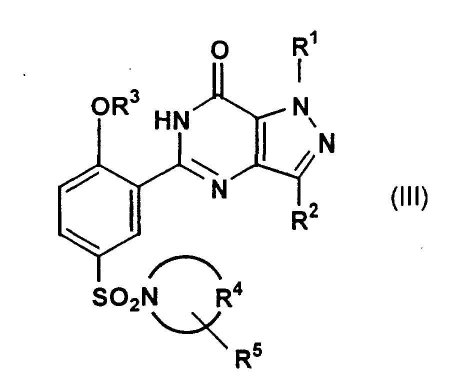 (where R¹ is a hydrogen atom, an alkyl group having 1 - 3 carbon atoms, a cycloalkyl group having 3 - 5 carbon atoms or a perfluoroalkyl group having 1 - 3 carbon atoms; R² is selected from the group consisting of a hydrogen atom, an alkyl group having 1 - 6 carbon atoms which is optionally substituted by a hydroxyl group, an alkoxy group having 1 - 3 carbon atoms or an cycloalkyl group having 3 - 6 carbon atoms and a perfluoroalkyl group having 1 - 3 carbon atoms; R³ is selected from the group consisting of an alkyl group having 1 - 6 carbon atoms, an alkenyl group having 3 - 6 carbon atoms, an alkynyl group having 3 - 6 carbon atoms, a cycloalkyl group having 3 - 7 carbon atoms, a perfluoroakyl group having 1 - 6 carbon atoms and an alkyl group having 1 - 6 carbon atoms which is optionally substituted by an cycloalkyl group having 1 - 3 carbon atoms (hereinafter, abbreviated as C₃₋₆ cycloalkyl); R⁴ forms a group selected from the group consisting of pyrrolidinyl, piperidino, morpholino and 4-N-(R⁶)-piperazinyl together with a nitrogen atom to which R⁴ is bonded; R⁵ is selected from the group consisting of a hydrogen atom, an alkyl group having 1 - 4 carbon atoms, an alkoxy group having 1 - 3 carbon atoms, NR⁷R⁸ and CONR⁷R⁸; R⁶ is selected from the group consisting of a hydrogen atom, an alkyl group having 1 - 6 carbon atoms, (C₁₋₃ alkoxy)-alkyl group having 2 - 6 carbon atoms, hydroxy-alkyl group having 2 - 6 carbon atoms, (R⁷R⁸N)-alkyl group having 2 - 6 carbon atoms, (R⁷R⁸NCO)-alkyl group having 1 - 6 carbon atoms, CONR⁷R⁸, CSNR⁷R⁸ and C(NH)NR⁷R⁸; and R⁷ and R⁸ each independently is selected from the group consisting of a hydrogen atom, an alkyl group having 1 - 4 carbon atoms, (C₁₋₃ alkoxy)- alkyl group having 2 - 4 carbon atoms and hydroxy-alkyl group having 2 - 4 carbon atoms).

A preferred compound is sildenafil.

The seventh aspect of the present invention is a pharmaceutical composition for intraurethral administration for preventing or treating erectile dysfunction wherein the composition contains at least one of the compounds represented by the above formula (III) and salts or solvates thereof as an effective component.

The eighth aspect of the present invention is a pharmaceutical composition for intraurethral administration for preventing or treating female sexual dysfunction wherein the composition contains at least one of the compounds represented by the above formula (III) and salts or solvates thereof as an effective component.

The ninth aspect of the present invention is a pharmaceutical composition for intraurethral administration, especially a pharmaceutical composition for intraurethral administration for preventing or treating erectile dysfunction or for preventing or treating female sexual dysfunction, wherein the composition contains at least one of 5-[2-ethoxy-5-(4-methylpiperazin-1-ylsulfonyl)phenyl]-1-methyl-3-propyl-6,7-dihydro-1H-pyrazolo[4,3-d]pyrimidin-7-one and salts thereof as an effective component.

The dosage forms of the pharmaceutical compositions of the present invention include a preferably a urethral suppository, a urethral cream, a urethral ointment, a urethral paste, a urethral gel, urethral suspension, a urethral dispersion, a urethral liquid, or a urethral pellet. It is preferred that the drug of the present invention is formulated as a solid that melts at body temperature or coated on the surface of an insert.

The tenth aspect of the present invention is a pharmaceutical composition for intraurethral administration containing at least one of the compounds mentioned in the following publications and salts or solvates thereof as an effective component (JP-A-52-100479, JP-A-53-103497, JP-A-61-236778, JP-A-62-030796, JP-A-62-030782, JP-A-63-196585, JP-A-2-088577, JP-A-2-001463, JP-A-1-311067, JP-A-3-145466, JP-A-2-223580, JP-A-2-042079, JP-A-2-056484, JP-A-2-040388, JP-A-3-261785, JP-A-2-193983, International Publication WO 93/012,095, JP-A-2-295977, JP-A-2-295978, International Publication WO 91/19,717, International Publication WO 93/06,104, International Publication WO 93/07,149, International Publication WO 93/07124, JP-A-7-010843, JP-A-5-222000, JP-A-5-301857, JP-A-6-192235, JP-A-8-099962, US Patent No. 5,436,233, US Patent No. 5,439,895, JP-A-7-188214, International Publication WO 94/00,453, US Patent No. 5,294,612, International Publication WO 94/05,561, International Publication WO 94/19,351, International Publication WO 94/29,277, JP-A-7-061949, JP-A-7-089958, International Publication WO 95/06,648, International Publication WO 96/26,940, JP-A-7-126255, JP-A-8-188563, JP-A-7-267961, JP-A-7-285993, JP-A-7-330777, JP-A-8-143571, International Publication WO 96/05,176, JP-A-8-059681, International Publication WO 96/28,446, JP-A-8-104679, US Patent No. 5,525,604, JP-A-8-253457, JP-A-8-231545, JP-A-8-231546, US Patent No. 5,541,187, International Publication WO 96/28,429, International Publication WO 96/28,448, JP-A-8-269060, JP-A-8-269059, International Publication WO 96/32,379, JP-A-8-253484, JP-A-8-311035, JP-A-9-077764, International Publication WO 97/24,334, International Publication WO 98/06,722, International Publication WO 98/08,848, European Patent Publication No. 636,626, International Publication WO 97/19,947, International Publication WO 97/19,978, International Publication WO 96/32,003, International Publication WO 97/03,675, International Publication WO 97/03,985, JP-A-9-124648, International Publication WO 97/43,287, JP-A-10-67682, International Publication WO 98/16,224, International Publication WO 98/16,521, German Laid-Open Patent Publication No. 19642319, German Laid-Open Patent Publication No. 19642320, German Laid-Open Patent Publication No. 19642322, International Publication WO 98/16,507, International Publication WO 98/17,668, International Publication WO 98/14,448, International Publication WO 98/15,530, International Publication WO 98/23,597, JP-A-10-120681, JP-A-10-120680, JP-A-10-175972, JP-A-10-182459, International Publication WO 98/31,674, International Publication WO 98/32,755, International Publication WO 98/40,384, German Laid-Open Patent Publication No. 19709126, International Publication WO 98/38,168, Japanese Laid-Open Patent Publication Hei-10/245,338, International Publication WO 98/30,209, JP-A-10-298062, JP-A-10-081688, International Publication WO98/49,166, US Patent No. 5,824,683, International Publication WO 99/00,373, International Publication WO 99/00,372, International Publication WO 99/00,359, U.S. Patent No. 5,854,419, JP-A-11-005794, International Publication WO 99/02,161, European Patent Publication No. 911333, International Publication WO 99/24,433, International Publication WO 99/28,325, International Publication WO 99/21,831, German Laid-Open Patent Publication No. 19750085, International Publication WO 99/35,142, International Publication WO 99/31,065, International Publication WO 99/32,460, International Publication WO 99/42,444, International Publication WO 99/43,674, International Publication WO 99/21,558, German Laid-Open Patent Publication No. 19812462, International Publication WO 99/43,769, International Publication WO 99/42,452, International Publication WO 99/51,574, International Publication WO 99/54,284, International Publication WO 99/54,333, German Laid-Open Patent Publication No. 19819023, International Publication WO 99/28,319; these publications being incorporated herein by reference.)

The eleventh aspect of the present invention is a pharmaceutical composition for intraurethral administration for preventing or treating erectile dysfunction or for preventing or treating female sexual dysfunction wherein the composition contains at least one of the compounds having a cGMP-PDE inhibitory action mentioned in the publications referred to in the tenth aspect of the present invention or a salt or a solvate thereof as an effective component.

### [Embodiment Mode for carrying out the Invention]

The present invention will now be described below in detail.

The compounds represented by the formula (I) which are effective components of the pharmaceutical composition for intraurethral administration are compounds which have extremely high enzyme selectivity and remarkable PDE type V inhibitory action mentioned in International Publication WO 97/45,427.

The compounds represented by the formula (I), or salts or solvates thereof mentioned herein can be produced by use of the method mentioned in International Publication WO 97/45,427 or methods similar thereto.

The method for measuring the PDE inhibitory activity of the compounds described herein is disclosed in International Publication WO 97/45,427.

The compounds represented by the formula (III) described herein are disclosed as pyrazolopyrimidinones which are effective for impotence by oral administration in International Publication WO 94/28,902 and the compounds and pharmaceutically acceptable salts, the method for preparing, and the method for measuring the cGMP-PDE inhibitory activity of the compounds or salts thereof are disclosed in European Patent Publication No. 463756 and European Patent Publication No. 526004.

These publications are herein incorporated by reference.

The compounds having a cGMP-PDE inhibitory action which can be provided in the present invention includes the compounds represented by the formula (I) or (III) above and the compounds having a cGMP-PDE inhibitory action disclosed in the publications referred to in the tenth aspect of the present invention described above. Further, the compounds having a cGMP-PDE inhibitory action which can be provided in the present invention also includes the compounds having a PDE type V inhibitory action as described below.
IC-351 (ICOS); 4-bromo-5-(pyridylmethylamino)-6-[3-(4-chlorophenyl)propyl]-3(2H)pyridazinone; 1-[4-[(1,3-benzodioxol-5-ylmethyl)amino]-6-chloro-2-quinazolinyl]-4-piperidine-carboxylic acid monosodium salt; (+)-cis-5,6a,7,9,9,9a-hexahydro-2-[4-(trifluoromethyl)-phenylmethyl-5-methyl-cyclopent-[4,5]imidazo[2,1-b]purin-4(3H)-one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6-carboxylate; 4-bromo-5-(3-pyridylmethylamino)-6-(3-(4-chlorophenyl)propoxy)-3-(2H)pyridazinone; 1-methyl-5-(5-morpholinoacetyl-2-n-propoxyphenyl)-3-n-propyl-1,6-dihydro-7H-pyrazolo-(4,3-d)pyrimidin-7-one; 1-[4-[(1,3-benzodioxol-5-ylmethyl)amino]-6-chloro-2-quinazolinyl]-4-pipedinecarboxylic acid monosodium salt; Pharmprojects No. 4516 (Glaxo Wellcome); Pharmprojects No. 5051 (Bayer); Pharmprojects No. 5064 (Kyowa Hakko; WO 96/26940); Pharmprojects No. 5069 (Schering Plough); GF-196960 (Glaxo Wellcome); Pharmprojects No.5890 (Merck); Pharmprojects No. 4999 (Eisai); Pharmprojects No. 5410 (Fujisawa); E-4010 (Eisai); E-4021 (Eisai); Sch-51866 (Schering Plough); Sch-59498 (Schering Plough); Bay-38-9456 (Bayer); EMD-86239 (uMerck); E-8010 (Eisai); SR 265579 (Chino in Pharmchem Works, Sanofi Rech); UK-114542 (Pfizer); UK-313794 (Pfizer); UK-343664 (Pfizer); (These compounds are herein incorporated by reference).

The pharmaceutical composition of the present invention may contain at least one of the compounds having a cGMP-PDE inhibitory action, more preferably the compounds represented by the formula (I), which may be used in combination with a pharmaceutically acceptable additive or additives. More specifically, oils and fats (e.g., lanoline, vaseline, hard fat (Witepsol and Pharmasole), and polyethylene glycols) as a base material, emulsifiers (nonionic surfactants (polyoxyethylene hydrogenated castor oil, polyoxyethylene alkyl ether, polyoxyethylene polyoxypropylene glycol, isopropyl myristate, glycerol monostearate, sorbitan monostearate, etc.), anionic surfactants (alkyl sulfates, polyoxyethylene alkyl ether sulfates, N-acylamino acid or salts thereof, polyoxyethylene alkyl ether phosphates, etc.), cationic surfactants (alkylammonium salts, alkylbenzylammonium salts, etc.), amphoteric surfactants (betaine acetate, lecithin, etc.)), thickeners (e.g., acrylic acid copolymer (cyclofibroin acylate copolymer, etc.), fructose, carnauba wax, agar powder, glycerol, magnesium aluminum silicate, purified gelatin, hydroxyethylcellulose, hydroxypropylcellulose, propylene glycol, sodium polyacrylate, carboxyvinyl polymer (Carbopol (produced by Goodrich Chemical) , Hivis Wako (produced by Wako Pure Chemical Industries), macrogol), adhesives (e.g., sodium alginate, xanthan gum, magnesium aluminum silicate, sodium chondroitin sulfate, concentrated glycerol, hydroxyethylcellulose, hydroxypropylcellulose, macrogol, and methylcellulose, liquid paraffin), dispersants (e.g., Acacia, propylene glycol alginate, carboxyvinyl polymer, dried egg white, sodium citrate, magnesium silicate, crystalline cellulose, hydrogenated oils, sucrose fatty acid ester, magnesium stearate, zinc stearate, sodium cetylsulfate, sorbitan fatty acid ester, D-sorbitol, soybean oil, dextrin, corn starch, potato starch, and lactose, saccharides (disaccharides such as sucrose and maltose, and sugar alcohols such as mannitol and sorbitol), hydroxyethylcellulose, methylcellulose, propylene glycol, polysorbate, macrogol, and egg yolk oil), solubilizers (e.g., tartaric acid, citric acid, lactic acid, L-aspartic acid, L-arginine, sodium benzoate, sodium citrate, glycerol, propylene glycol, sucrose fatty acid ester, polyoxyl stearate, purified soybean lecithin, sorbitan fatty acid ester, soybean oil, hydroxypropylcellulose, propylene fatty acid ester, polyoxyethylene polyoxypropylene alkyl ether, polyoxyethylene hydrogenated castor oil, polyoxyethylene polyoxypropylene glycol polysorbate, macrogol, maleic acid, and liquid paraffin), stabilizers (e.g., amino acids or salts (basic amino acids such as arginine and lysine, acidic amino acids such as glutamic acid and aspartic acid), xanthan gum, etc.), absorption accelerators (e.g., sodium glycolate, sodium edetate, sodium caprylate, acyl carnitines, and limonene), solubilizing agents (e.g., polyoxyethylene hydrogenated castor oil, polysorbate 80, polyethylene glycol, phospholipid cholesterol, and triethanolamine), and other commonly used suitable additives or solvents such as suspending or emulsifying agents may be combined appropriately with the compounds which form the effective component of the composition of the present invention into various dosage forms suitable for intraurethral administration.

Further, disintegrators (e.g., corn starch and carboxymethyl cellulose), antiseptics (benzalkonium chloride, paraoxybenzoate), isotonic agents (e.g., glycerol, sodium chloride, calcium chloride, mannitol, and glucose), pH adjusting agent (sodium hydroxide, potassium hydroxide, sodium carbonate, hydrochloric acid, sulfuric acid, and buffers such as phospate buffer), antioxidants (e.g., ascorbic acid, butyl hydroxyanisole (BHA), propyl gallate and dl-α-tocopherol), buffering agents, preservatives (e.g., parabens, benzyl alcohol and benzalkonium chloride), fragrances (e.g., vanillin, 1-menthol and rose oil), excipients (e.g., lactose, sucrose, mannitol, crystalline cellulose and silicic acid), binders (e.g., crystalline cellulose, sugars (e.g., mannitol, sucrose, sorbitol, erythritol and xylitol), dextrin, hydroxypropyl cellulose (HPC), hydroxymethyl cellulose (HPMC), polyvinyl pyrrolidone (PVP) and macrogol), lubricants (e.g., magnesium stearate, calcium stearate and talc), coloring agents, flavoring agents, and the like may be added. The dosage forms include suppositories, creams, ointments, pastes, gels, suspensions, dispersions, liquid agents, pellets and the like that are suitable for intraurethral administration.

The intraurethral suppository has a shape suitable for insertion into urethra, usually having an elongate pencil-like shape with one end thereof is relatively thin and is used with a length of 7 to 14 cm long for males or 5 to 7 cm long for females. The solid or semi-solid pharmaceutical composition is made to have a size suitable for intraurethral administration. This volume may be controlled appropriately and a pharmaceutical composition of 1 cm or less in length may be formulated.

The composition of the present invention when it is necessary is administered into urethra in a predetermined amount directly or using a flexible tube, a squeeze bottle or a device that can be inserted into the urethra.

Dose of the pharmaceutical composition of the present invention is 1 to 1,000 mg, preferably 5 to 500 mg, more preferably 10 to 300 mg, and particularly preferably 20 to 200 mg per one dosage although that may be appropriately increased or decreased depending upon a patient. It is also possible that the dose is administered at a time or on a divided manner (2 to 6 times).

The volume of the drug of the present invention per administration unit is 6 ml or less, preferably 1 to 3 ml and suitable volume is selected depending upon the allowable volume of the urethra of an individual patient.

The composition of the present invention is expected to exhibit its effect in a few minutes to about 30 minutes after the administration. The composition of the present invention can provide a sustained erection preparatory state where natural erection is possible upon a sexual stimulation. It also has a potential for improving the symptom of female sexual dysfunction, for example, lack of sexual sensibility (excited state) (such as tactile disability, lack of clitoris sensibility, dryness of vagina, acraturesis or coital pain).

The drug of the present invention may be formulated into a dosage form such as a suitable solid (base material) that melts at body temperature as described above or a dosage form coating uniformly applied on a suitable insert. The "insert" may be of any material that is pharmaceutically acceptable. It may be hard. However, taking comfortableness into consideration, it is desirably a soft and flexible device that has a hardness only sufficient for facilitating its insertion. More specifically, it may be made of various natural rubbers or synthetic rubbers or polymer materials that are pharmaceutically acceptable, such as natural rubber, silicone rubber, ethylene-vinyl acetate (EVA) copolymer, polyethylene, polypropylene, polycarbonate, polyester, polyurethane, and polyisobutylene polymer.

As a substitute for the above-mentioned intraurethral administration of cGMP-PDE inhibitor, mention may be made of needleless injection of cGMP-PDE inhibitor. No disclosure at all of such an administration form of cGMP-PDE inhibitor is found in the above-mentioned prior art. The "needleless injection" referred to herein is an injection method in which a drug is passed through the skin, without using a needle for injection but by means of pressure, and the method my be roughly grouped into a type in which powder of a drug is directly administered (hereinafter, described as "powder type") and a type in which solution of a drug is administered by applying pressure thereto (hereinafter, described as "solution type").

Such a needleless injection can be practiced based on the following explanation.

The pharmaceutical composition for needleless injection may contain at least one of cGMP-PDE inhibitors, for example, the compounds represented by the above-mentioned formula (I) or (III), the compounds having a cGMP-PDE inhibitory action described in the publications referred to in the tenth aspect of the present invention, and the above-mentioned compounds having an inhibitory action to PDE type V and is prepared into a form that can be used for needleless injection and put into use. The composition of the present invention may be used optionally in combination of the compounds having a cGMP-PDE inhibitory action and pharmaceutically acceptable additives or may be prepared without using any additive so as to comprise substantially 100% of the active compound.

First, the "powder type" is explained.

As the needleless injection device for administration of "powder type", known devices may be used which are disclosed in, for example, International Publication WO 94/24,236, International Publication WO 96/04,947, International Publication WO 96/25,190, International Publication WO 96/12,513, International Publication WO 96/20,022, or International Publication WO 97/34,652. These also disclose various conditions upon administration. (These publications are herein incorporated by reference.)

The powder as the form that can be used in the "powder type" is prepared, for example, by preparing the active compound into fine powder by a spray-drying method, applying pressure (0.5 to 12 ton/inch) in order to further increase the density to solidify it, then pulverizing the pressured product by a usual pulverizing means such as a roller mill, a ball mill, a jet mill, an SWECO mill, an attritor mill, a hammer mill, or a planet ball mill, and classifying the particle size. By this treatment, the powder to be administered is prepared to have a necessary particle size and a particle density. The particle size is 0.1 to 250 µm, preferably 0.1 to 150 µm, and more preferably 10 to 60 µm. The density of the particle is in the range of 0.1 to 25 g/cm³, preferably 0.5 to 3.0 g/cm³, and more preferably 0.8 to 1.5 g/cm³. In preparing the above-mentioned powder, pharmaceutically acceptable additives may be added. When the powder is prepared by mixing the active compound and the additive, the total amount of the additive is preferably less than 75%, more preferably less than 50%, of the volume of particles. The addition of the additive powder may be performed at any of the time of; a) preparing a solution for a spraying-drying method, b) compression after the spraying-drying, or c) granulating after the pulverization. On the method for preparing the above-mentioned powder, reference is made of International Publication WO 97/48,485 (which is herein incorporated by reference). The particles can be provided with a suitable coating so as to have a sustained efficacy.

The velocity of injection to the administration site may be 200 to 2,500 m/sec. To transdermally inject the powder, the velocity of injection is preferably 50 to 1,500 m/sec and more preferably 750 to 1,000 m/sec.

As a method for increasing the velocity at the time of injection, a method of utilizing pressure by means of a gas (nitrogen gas, helium gas, carbon dioxide gas or the like) or a spring. The gas for pressurization for use in administration is preferably helium in view of applicability to inert, sterile form, permeability through the skin, and the like. To increase the velocity of shock wave generated by the pressure, the gas is desirably lighter than air. The gas pressure in this case is set to 20 to 300 atm, preferably 20 to 100 atm and the velocity of the generated supersonic shock wave is in the range of Mach 1 to 8, preferably 1 to 3.

The pharmaceutical composition of the "powder type" for needleless injection is administered in an administration region of preferably 20 to 30 mm in diameter.

Next, "solution type" will be explained below.

The form that can be used for "solution type" include medicinal liquids such as solution and suspension. In the case of suspension, it is preferred to use powder of the above-mentioned particle diameter. As the needleless injection device for administration in the form of "solution type", known devices may be used, as disclosed in the Japanese Patent Translation Publication Hei-7-509161, JP-A-63-315063, or JP-A-9-099082 (the publications being herein incorporated by reference). These publications also disclose various conditions upon administration, for example, pressure and velocity. As for the administration region of "solution type", the diameter of pore for spraying is 0.3 mm or less, preferably about 0.1 to 0.2 mm.

The pharmaceutical composition for needleless injection is administered to penis. When the "powder type" is administered to the penis, it is administered preferably to the mucous membrane of the glans and it is preferred that it be administered usually at lower energy than in the case where it is administered transdermally, for example, at a low velocity or pressure reduced to 90 to 10%.

The dose of a drug for needleless injection is 0.1 to 100 mg, preferably 0.3 to 50 mg, more preferably 0.5 to 20 mg, and particularly preferably 0.5 to 6 mg per administration unit although that may be appropriately increased or decreased depending upon patient. It is also possible that the dose is administered at a time or on a divided manner (2 to 6 times). In the case of "powder type", the dose is 0.5 to 20 mg, preferably 0.5 to 6 mg. In the case of "solution type", the dose of the administration liquid is adjusted to 0.1 to 0.5 ml.

The composition for needleless injection is expected to exhibit its effect in a few minutes to about 30 minutes after the administration. It can provide a sustained erection preparatory state where natural erection is possible upon a sexual stimulation.

Use of an administration method by needleless injection, in contrast to the injection into the corpus cavernosum using a needle, the compositions can be administered by the patient himself with reduced burden on the both the patient and the doctor and are free from potential side effects such as pain or corporal fibrosis of penis and the fear of infection accompanied by the injection.

The pharmaceutical composition for needleless injection may be transdermally or transmucously administered to a site other than penis. More specifically, transdermal administration includes administration to skin surface (inclusive of intracutaneous, subcutaneous, intramuscular and the like administrations), transmucous administration includes gum in the buccal cavity, bucca or mucous membranes of palate, vagina, rectum, nose or eye. In this case, the dose of the drug is larger than that for administration to penis.

### [Formulation Example]

Next, formulation examples of the pharmaceutical composition for intraurethral administration according to the present invention will be shown below. However, the present invention is by no means limited thereto.

### (Formulation Example 1 Pellets for urethra)

Pellets of 1 mm in diameter and 7 mm in length were prepared from 10 mg of the compound 1 described later.

### (Formulation Example 2 Insert for urethra)

EVA (ethylene-vinyl acetate copolymer, 24% VA) rod of 3 mm in diameter and about 10 cm in length was rounded and smoothened on one end thereof. A gel-like coating composition containing 9 mg of 1% tartaric acid aqueous solution, 1 mg of propylene glycol, 0.2 mg of hydroxycellulose and 20 mg of compound 1 and the like was prepared and the rod was dipped therein to coat it such that a total charge amount on the shaft portion of the rod was 500 mg. The moisture on the coated rod was evaporated. The amount of the compound was about 2 mg per administration unit.

| (Formulation Example 3 Urethral suppository) | |
|---|---|
| Compound 1 | 60.0 g |
| Whitepsol™ H15 | 187.0 g |

187.0 g of Whitepsol™ H15 was heated at 120°C and molten. A portion of this (about 20 g) was taken and sufficiently kneaded together with 60.0 g of compound 1 in an agate mortar while adding the remaining Whitepsol™ H15 slowly and the entire composition was uniformly kneaded. Thereafter, the temperature was lowered to 40°C and the resulting composition was packed into a polyethylene tube of 3 mm in inner diameter and cooled to solidify it, and then the solid was cut to 3.5 cm long.

| (Formulation Example 4 Urethral Suppository) | |
|---|---|
| Compound 1 | 58.2 g |
| Polyethylene glycol 1500 | 180.0 g |
| Polyethylene glycol 4000 | 720.0 g |

58.2 g of compound 1 was sufficiently ground in a mortar to obtain very fine powder. Then, 180 g of polyethylene glycol 1500 and 720 g of polyethylene glycol 4000 were heated at 120°C and molten. These were mixed and then uniformly kneaded. Thereafter, the temperature was lowered to 40°C and the resulting composition was packed in a polyethylene tube of 3 mm in inner diameter and cooled to solidify it, and then the solid was cut to 7.0 cm long.

| (Formulation Example 5 Urethral Gel) | |
|---|---|
| Compound 1 | 30.0 g |
| Tartaric acid | 3.0 g |
| Hydroxypropylmethylcellulose 2208 (apparent viscosity 4000) | 2.0 g |
| Methylparaben | 0.1 g |
| Propylparaben | 0.1 g |

The above components were weighed and the components except for hydroxypropylmethylcellulose 2208 were uniformly kneaded. To this was added 40 to 50 g of purified water and mixed. Thereafter, the resulting liquid was stirred and hydroxypropylmethylcellulose 2208 was added thereto portionwise. Purified water was added with the weight of 100g in total.

| (Formulation Example 6 Urethral Gel) | |
|---|---|
| Sildenafil | 10.0 g |
| Tartaric acid | 1.0 g |
| Hydroxypropylmethylcellulose 2208(apparent viscosity 4000) | 2.0 g |
| Methylparaben | 0.1 g |
| Propylparaben | 0.1 g |

The above components were weighed and the components except for hydroxypropylmethylcellulose 2208 were uniformly kneaded. To this was added about 70 g of purified water and mixed. Thereafter, hydroxypropylmethylcellulose 2208 was added thereto portionwise while stirring the liquid. Purified water was added to the mixture with the weight of 100g in total.

| (Formulation Example 7 Urethral Cream) | |
|---|---|
| Compound 1 | 1.2 g |
| Propylene glycol | 12 g |
| Stearyl alcohol | 20 g |
| White vaseline | 25 g |
| HCO-60 | 4 g |
| Glycerol monostearate | 1 g |
| Methylparaben | 0.1 g |
| Propylparaben | 0.1 g |

Stearyl alcohol, white vaseline, HCO-60 and glycerol monostearate were each heated at about 75°C to dissolve. Separately, compound 1, methylparaben and propylparaben were dissolved in propylene glycol and 20 g of purified water was added to the solution and then the mixture was heated at about 75°C. This was added to the above mixture, which was then stirred to form an emulsion. This was cooled slowly while stirring it.

| (Formulation Example 8 Urethral Aqueous Gel) | |
|---|---|
| Compound 1 | 4 g |
| Propylene glycol | 10 g |
| Hivis Wako 105 (Wako Pure Chemical Industries) | 2 g |
| Diethylamine | 2 g |
| Purified water to make | 100 g |

Propylene glycol solution of compound 1 and Hivis Wako 105 were added to purified water and mixed with stirring. Then, diethylamine was added to the mixture to increase the viscosity to prepare an aqueous gel.

| (Formulation Example 9 urethral Liquid) | |
|---|---|
| Compound 1 | 10 g |
| Tartaric acid | 1 g |
| Benzalkonium chloride | 30 mg |

The above components were weighed and then purified water was added thereto to make total amount 100 ml to prepare a urethral liquid.

### [Test Example]

Next, one example of the pharmaceutical composition of the present invention will be described on its pharmacological action. However, the present invention is by no means limited thereby.

### (Experiment)

A mongrel dog (weighing 7 kg) was anethesized with intravenous administration of 25 mg/kg of sodium pentobarbital and the anesthesia was maintained with continuous intravenous administration of 5 mg/ml/hr during and after the experiment. 21G needle equipped at the tip of a polyethylene tube was punctured into the corpus cavernosum of penis and the intracavernous pressure was measured using a pressure transducer.

The test drug was prepared according to Formulation Example 9 and diluted to make 1 mg/0.05 ml and 5 mg/0.05 ml.

A tube (SP45, Natsume Seisakusho) was inserted to a depth of about 15 cm in a urethra and the test drug was administered in the urethra in a volume of 0.05 ml/kg body weight for 15 seconds. At 5 minutes, 15 minutes and 30 minutes after the administration of the drug in the urethra, 1 µg/0.2 ml of SNP (sodium nitroprusside) was topically administered into the corpus cavernosum of penis for 15 seconds using a 25G needle, and the intracavernous pressure was determined. An increase in intracavernous pressure induced by the same amount of SNP before the administration of the test drug was taken as 100% (control) and an increase in intracavernous pressure after administering each dose was measured and the results are shown in Table 1 in %.

Test drug: 9-bromo-2-(3-hydroxypropyloxy)-5-(3-pyridylmethyl)-4H-pyrido[3,2,1-jk]carbazol-4-one methanesulfonic acid salt (compound 1)

**Table 1**

| Intracavernous Pressure Increasing Action | | |
|---|---|---|
| | Dose (mg/Kg) (mg/Kg) | Increase in inner pressure of corpus cavernosum (%) (after 5 minutes) |
| Compound 1 | 1 | 181 |
| Compound 1 | 5 | 215 |

Five minutes after administration, the test drug enhanced an increase in intracavernous pressure induced by SNP injection. Thirty minutes after administration, the test drug did not enhance increases in intracavernous pressure at each dose. That is, by the intraurethral administration, quick absorption was observed without any influence by first pass effect and an appropriate duration of the action was obtained. On this occasion, no side effect on hemodynamics including systemic hypotension was observed.

From the foregoing, the compounds represented by the above formula (I) and having remarkable inhibitory action on PDE type V and high selectivity in enzyme inhibition are absorbed quickly by intraurethral administration and augment increasing the action on the inner pressure of the corpus cavernosum of penis in a dog.

Therefore, the composition of the present invention provides quick absorption and appropriate efficacy duration of the action but causing no prolonged erection symptom. When it is used for erectile dysfunction, it is possible to sustain the erection preparation state where natural erection is possible upon a sexual stimulation. Further, the composition of the present invention is not affected by first pass effect of the digestive tract and the liver and are free from the potential side effects in the digestive organs. As compared with injection to the corpus cavernosum or conventional intraurethrally administered preparations, it has no side effect such as pain of penis and corporal fibrosis and that it can be injected by himself/herself whereby burden of patients and doctors is little. As compared with oral administration, absorption of the composition of the present invention is not affected by food as is known with sildenafil. It causes no decrease of systemic blood pressure and can be used as a composition having less side effect and high safety and is more useful and more simpler than administrations in other routes.

### [Industrial Applicability]

The compound of the present invention having an inhibitory action to PDE-V, i.e., the active component of the pharmaceutical composition for intraurethral administration, has extremely high selectivity to PDE type V enzyme inhibition and has a weak blood pressure lowering action. It has extremely low toxicity and less side effects such as prolonged erection symptom. Therefore, the pharmaceutical composition of the present invention is useful clinically and as a drug for animals. It is expected to have positive effects in prevention or treatment of erectile dysfunction or female sexual dysfunction.

The composition for intraurethral administration of the present invention can provide quick absorption and appropriate duration of the action without causing any prolonged erection symptom. When it is used for erectile dysfunction, it is possible to sustain the erection preparation state where natural erection is possible upon a sexual stimulation. Further, the composition of the present invention is not affected by first pass effect of the digestive tract and the liver and are free from the potential side effects in the digestive organs. As compared with injection to the corpus cavernosum or conventional intraurethrally administration, it has no side effect such as pain of penis and corporal fibrosis and it can be injected by a patient himself/herself whereby burden of patients and doctors is little. As compared with peroral administration, absorption of the composition of the present invention is not affected by food as is known in sildenafil. It causes no decrease of systemic blood pressure and can be used as a composition having less side effect and high safety and is simpler and more useful than administrations in other routes.

The pharmaceutical composition of the present invention may contain absorption accelerators for accelerating its absorption from the urethra to the corpus cavernosum or a drug delivery system such as iontophoresis.

## Claims

1. Pharmaceutical compositions for intraurethral administration wherein the composition contains at least one of the compounds represented by the following formula (I), and salts and selected from the group consisting of solvates thereof as an effective component: 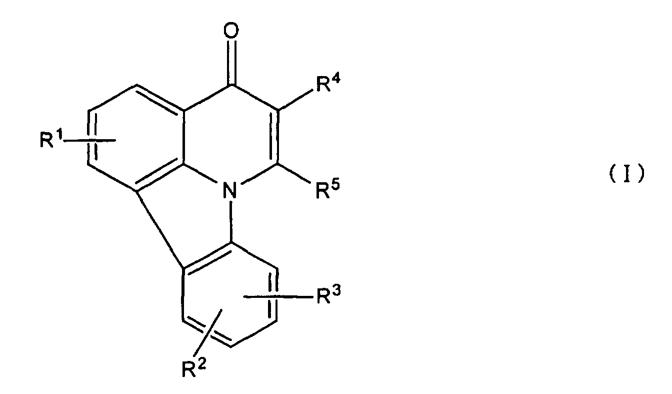 (where R⁵ represents a hydrogen atom, a halogen atom, a cyano group, an optionally protected carboxyl group, an optionally protected carboxymethyl group, an alkoxycarbonyl group having 1 to 4 carbon atoms, a carbamoyl group, an acetylamino group, a 3-carboxy-1-propenyl group, a 2-hydroxypentyloxy group, a 2,2-diethoxyethoxy group, an optionally protected hydroxyl group, an optionally protected mercapto group, a straight- or branched-chain alkanoyloxy group having 1 to 4 carbon atoms, a carbonyloxy group substituted with a phenyl group or a pyridyl group, a straight- or branched-chain alkyl group having 1 to 4 carbon atoms which may be substituted with one hydroxyl group, an amino group which may be mono- or disubstituted with an alkyl group having 1 to 4 carbon atoms, an alkylthio group having 1 to 3 carbon atoms which may be monosubstituted with any group selected from the group consisting of a hydroxyl group, a carboxyl group, a phenyl group and a pyridyl group, or represented by the following formula (II):
- O - (CH₂)ₙ - Z (II)
(where Z is selected from the group consisting of a hydrogen atom, a carboxyl group, an alkoxy group having 1 or 2 carbon atoms which may be substituted with one hydroxyl group, an alkoxycarbonyl group having 1 to 6 carbon atoms, a carbamoyl group which may be mono- or disubstituted with a hydroxymethyl group or an alkyl group having 1 or 2 carbon atoms, an alkanoyl group having 1 to 4 carbon atoms which may be substituted with one hydroxyl group or mercapto group, a piperidinylcarbonyl group which may be substituted with one carboxyl group or alkoxycarbonyl group having 1 or 2 carbon atoms, a morpholylcarbonyl group, a hydroxyl group, a mercapto group, an amino group, a phenyl group, a pyridyl group which may be monosubstituted with a hydroxymethyl group or an acetoxymethyl group or an alkyl group having 1 to 4 carbon atoms or an alkoxycarbonyl group having 1 or 2 carbon atoms, a pyrazinyl group, a pyrimidinyl group, a furyl group, a thienyl group, an oxadiazolyl group and a 4-methoxyphenoxy group; n is 1 to 6); R² is selected from the group consisting of a hydrogen atom, a halogen atom, an optionally protected hydroxyl group, an optionally protected mercapto group, an optionally protected amino group, a cyano group, a nitro group, a trifluoromethyl group, a trifluoromethoxy group, an optionally protected carboxyl group, a 4-morpholylacetyl group, a straight- or branched-chain alkanoyloxy group having 1 to 4 carbon atoms, a straight- or branched-chain alkanoyl group having 1 to 4 carbon atoms, a straight- or branched-chain alkyl group having 1 to 4 carbon atoms, an alkylthio group having 1 to 3 carbon atoms which may be monosubstituted with any group selected from the group consisting of a hydroxyl group, a carboxyl group, a phenyl group and a pyridyl group and a straight- or branched-chain alkoxy group having 1 to 4 carbon atoms which may be substituted with one alkoxycarbonyl group having 1 to 4 carbon atoms; R³ is selected from the group consisting of a hydrogen atom, a halogen atom, an optionally protected hydroxyl group and a straight- or branched-chain alkoxy group having 1 to 4 carbon atoms; R⁴ is selected from the group consisting of a hydrogen atom, a halogen atom, an optionally protected carboxyl group, a phenoxy group, an anilino group, a N-methylanilino group, a 4-morpholylcarbonyl group, an alkyl group having 1 or 2 carbon atoms which may be substituted with a cyclic alkyl group having 3 to 6 carbon atoms, a benzyl group which may be mono- or disubstituted in the phenyl portion with any group selected from the group consisting of a halogen atom, a hydroxyl group, a mercapto group, an alkoxy group having 1 or 2 carbon atoms, an alkylthio group having 1 or 2 carbon atoms, an alkoxycarbonyl group having 1 to 4 carbon atoms, an acetylamino group, a carboxyl group and an amino group, a pyridylmethyl group which may be substituted with an alkyl group having 1 to 4 carbon atoms, a morpholylmethyl group, a triazolylmethyl group, a furylmethyl group, a thienylmethyl group, a pyrimidinylmethyl group, a pyrazinylmethyl group, a pyrrolylmethyl group, an imidazolylmethyl group, a quinolylmethyl group, an indolylmethyl group, a naphthylmethyl group, a benzoyl group, an α-hydroxybenzyl group and an alkoxycarbonyl group having 1 or 2 carbon atoms; R⁵ represents a hydrogen atom or a methyl group; when R¹, R², R³ and R⁵ are a hydrogen atom at the same time, R⁴ is not a hydrogen atom, a benzyl group, a 4-diethylaminobenzyl group or a furylmethyl group.)

2. A pharmaceutical composition according to claim 1, which is used for prevention or treatment of erectile dysfunction or female sexual dysfunction.

3. A pharmaceutical composition according to claim 1 or 2, in which said dosage form is a urethral suppository, a urethral cream, a urethral ointment, a urethral paste, a urethral gel, a urethral suspension, a urethral dispersion, a urethral liquid, or a urethral pellet.

4. A pharmaceutical composition according to any one of claims 1 to 3, **characterized in that** a drug is formulated into a solid that melts at a body temperature or coated on a surface of an insert.

5. A method for preventing or treating erectile dysfunction, comprising intraurethrally administering at least one selected from the group consisting of the compounds represented by the formula (I) above, salts and solvates thereof.

6. Use of at least one selected from the group consisting of the compounds represented by the formula (I) above, salts and solvates thereof for producing a drug for preventing or treating erectile dysfunction.
